# EUROPEAN PATENT SPECIFICATION

(11) **EP 1 066 006 B1**
(45) Date of publication and mention of the grant of the patent: **21.05.2003**
(21) Application number: 99909463.4
(22) Date of filing: 17.03.1999
(51) Int. Cl.: A61F 13/15, A61F 13/50

(54) **MATERIAL LAMINATE FOR USE AS AN OUTER LAYER ON ABSORBENT PRODUCTS**
LAMINAT ZUR BENUTZUNG ALS ÄUSSERE SCHICHT AUF ABSORBIERENDEN PRODUKTEN
MATERIAU STRATIFIE SERVANT DE COUCHE EXTERIEURE SUR DES PRODUITS ABSORBANTS

(30) Priority: 27.03.1998 SE 9801038
(43) Date of publication of application: 10.01.2001
(73) Proprietor: SCA Hygiene Products AB, 405 03 Göteborg (SE)
(72) Inventor: HEDENBERG, Peter, S-414 77 Göteborg (SE); HANSSON, Roy, S-431 50 Mölndal (SE); TENNBY, Anders, S-416 53 Göteborg (SE); ELFSTRÖM, Anna-Carin, S-423 38 Torslanda (SE); KROOK, Fredrik, S-412 61 Göteborg (SE); GUSTAFSSON, Anders, S-427 35 Billdal (SE)
(74) Representative: Romare, Laila Anette
(86) International application number: SE9900407
(87) International publication number: WO99049825

(56) References cited:
- EP-A2- 0 685 214
- WO-A1-98/27904
- WO-A2-97/02133
- US-A- 4 333 979
- US-A- 4 446 189
- US-A- 5 580 418
- US-A- 5 688 258

## Description

### TECHNICAL FIELD:

The invention relates to a material laminate for use as an outer layer on absorbent products such as nappies, panty nappies, incontinence shields, sanitary towels, bandages or the like. The material laminate exhibits a planar dimension and a thickness direction perpendicular to the planar dimension and includes a first liquid-permeable fibrous material layer and a second liquid-permeable, porous and resilient material layer, with at least one of the material layers including thermoplastic material and the two material layers being mutually connected by the material laminate exhibiting bonding sites within which the thermoplastic material has been caused to at least partially soften or melt and thereby bond the two material layers together. The invention also relates to an absorbent product which includes the material laminate.

### BACKGROUND:

Absorbent products which are intended for single use normally exhibit a liquid-permeable outer layer which faces the body of the user when the product is used. Such an outer layer often consists of a nonwoven material, i.e. a fibre material in which the fibres included in it have been bound together in some other way than by means of weaving.

It is also known to arrange a liquid-transferring layer between the outer layer and an absorptive body which is included in the product. Such a liquid-transferring layer should have the ability to receive large quantities of liquid rapidly and spread the liquid and temporarily store it before it is absorbed by the underlying absorptive body. This is of great importance, especially in the case of today's thin, compressed absorptive bodies, which often have a high content of so-called superabsorbents. While such materials have a high absorptive capacity, they in many cases exhibit a rate of admission which is too low for managing instantaneously to absorb the large quantity of liquid which can be emitted over a few seconds in association with urination. A porous, relatively thick liquid-transferring layer, for example in the form of a fibre wad, a bound or unbound carded fibre layer, or some other type of fibre material, has a high capacity for receiving liquid instantaneously and can temporarily store the liquid until the absorptive body has had time to absorb it. This situation also applies to porous foam material. In order for the absorbent product to be able to receive repeated volumes of liquid, it is necessary for the liquid-transferring layer essentially to have time to be emptied of liquid between each wetting. In this connection, the porous structure of the liquid-transferring layer expediently interacts with a more compact and/or more hydrophilic absorptive body.

Examples of absorbent products which contain porous liquid-transferring layers are to be found in US-A-3,371,667, EP-A-0,312,118, EP-A-0,474,777, EP-A-685,214 and WO 97/02133.

A problem associated with the absorbent products which have been described is that the liquid-permeable outer layer materials often exhibit an effective median pore size which is less than the median pore size of the underlying recipient layer. In order to improve the liquid transfer between the outer layer and the liquid-transferring layer, EP-A-685,214 and WO 97/01233 have proposed that the two layers be bound to each other by the layers being melted together in a bonding pattern in the form of points or lines. However, a disadvantage of arranging a large number of bonds at a short distance from each other is that the surface material laminate loses volume and, as a result, pliancy and kindness to the skin. Furthermore, the bonds result in the material laminate becoming relatively stiff and, for this reason as well, less comfortable to wear in contact with the skin. As a result of the bonds decreasing the volume of the laminate, i.e. its thickness, the distance between the absorptive body of the product and the body of the user also decreases. This thereby increases the risk of liquid penetrating back out of the product and wetting the body of the user.

There thus remains a need for an improved surface material which exhibits good liquid-transferring ability and low rewetting and, at the same time, a high degree of pliancy, kindness to the skin and flexibility.

### BRIEF DESCRIPTION OF THE INVENTION:

The present invention provides a material laminate of the type specified in the introduction. The material laminate according to the invention is primarily distinguished by the fact that the bonding sites extend in the thickness direction of the material laminate, through the first material layer and at least through a part of the second material layer, and are arranged in two or more groups with at least two bonding sites in each group, with the greatest relative distance between two bonding sites which are located close to each other in a particular group being less than the shortest distance between the group and the neighbouring group which is located closest to it, as a result of which the material laminate exhibits bond-free areas between the bonding sites within each bonding group which have a higher density than bond-free areas of the material laminate which are located between the bonding groups, and wherein the shortest relative distance *x* between two groups of bonding sites, which two groups are situated adjacent to each other, is at least twice as great as the greatest relative distance *y* between two bonding sites which are arranged adjacent to each other within the groups.

Further distinctive features and embodiments are evident from the subsequent patent claims.

By means of arranging the bonds, in accordance with the invention, in a pattern which produces, from the bonds, limited areas of higher fibre density alternating with areas of lower fibre density, a material laminate which is of high bulk, pliancy and flexibility is obtained, at the same time as its ability to transfer liquid and its ability to store liquid temporarily are very good. In addition, a material laminate according to the invention is very airy and pleasant to wear against the skin and exhibits low rewetting.

### BRIEF DESCRIPTION OF THE FIGURES:

In that which follows, the invention will be described in more detail with reference to the figures which are shown on the attached drawings.

In this connection:
- Figure 1: shows a plane view of a material laminate according to the invention,
- Figure 2: shows a section along the line II-II through the material laminate in Figure 1,
- Figure 3: shows a first bonding pattern,
- Figure 4: shows a second bonding pattern,
- Figure 5: shows a third bonding pattern,
- Figure 6: shows a fourth bonding pattern,
- Figure 7: shows a fifth bonding pattern, and
- Figure 8: shows an incontinence shield with a material laminate according to the invention.

### DESCRIPTION OF EMBODIMENTS:

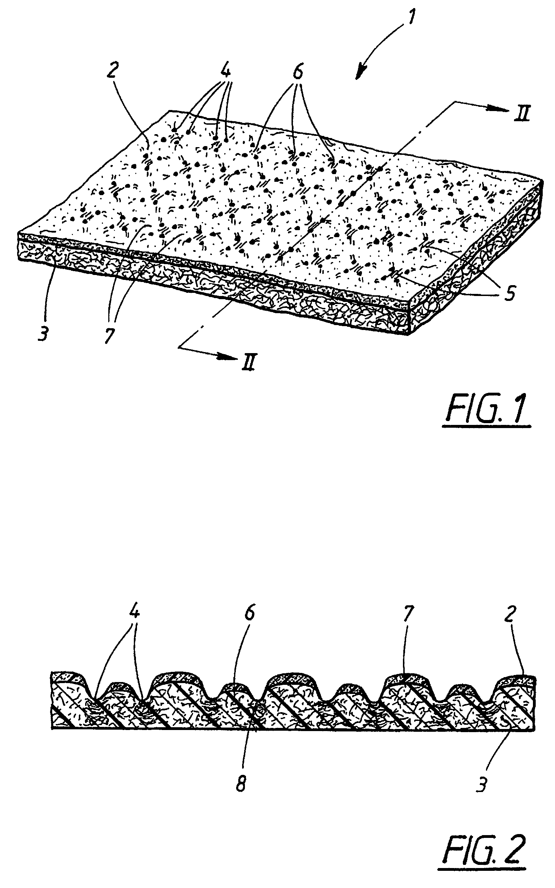

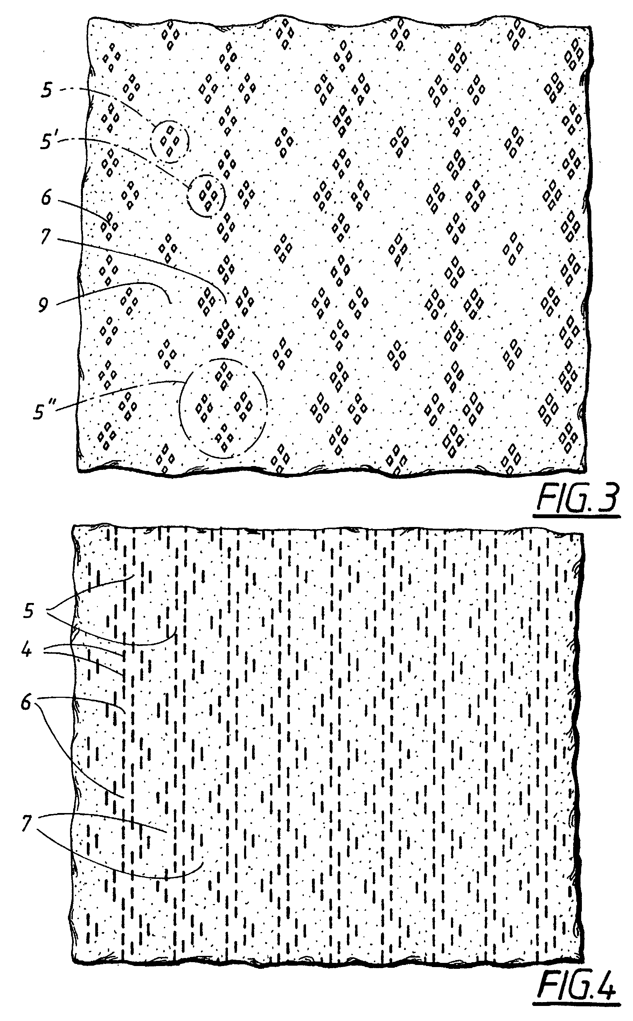

The material laminate 1 shown in Figures 1 and 2 includes a first material layer 2 and a second material layer 3. In this connection, the first material layer 2 expediently consists of a relatively thin nonwoven material.

Nonwoven materials can be produced by many different methods, for example by carding or spinning a fibre pile which is then bound. Furthermore, use can be made of the melt-blown technique in order to deposit short fibres in the form of a fibre mat. A number of different methods exist for binding the fibres in a nonwoven material. For example, different types of binding agent can be used. Furthermore, heat-meltable components in the material can be exploited for binding by means of ultrasound or by means of supplying heat. Other binding methods are needling and hydroentangling. Moreover, different binding methods can be combined with each other.

Since the material laminate is used as a liquid-permeable surface material on an absorbent product, the first material layer 2 is the layer which is intended to be facing a user of the product. In this connection, it is important that the surface of the first layer which is facing the user is smooth and soft.

The second material layer 3 is advantageously thicker than the first material layer 2 and consists of a porous, resilient fibre material having a thickness of 0.5-4 mm. The second material layer 3 serves as a liquid-transferring layer when the material laminate is arranged, as a surface material, on an absorbent product. In this connection, the second material layer 3 should have the ability to receive large quantities of liquid over a short period, spread the liquid in the plane of the material layer, convey the liquid onward to an absorptive body which is arranged under the material laminate 1 and, in addition, also be able temporarily to store liquid which the absorptive body has not had time to absorb.

Materials which are particularly well suited for use in the second material layer are synthetic fibre wads, carded fibre layers which are bound or unbound, or bulky nonwoven materials. A special type of fibre material which can be used is tow, which is understood to mean fibres which are in the main parallel, long or infinite, or fibre filaments which are present in the form of layers or strands. Porous, hydrophilic foam materials are another type of suitable material. The second material layer can furthermore consist of two or more layers of different materials or of the same type of material.

A composite nonwoven material, consisting of a first material layer 2 composed of a nonwoven material of synthetic fibres having a grammage of between 10 and 50 g/m² and a second material layer 3 composed of a wad of synthetic fibres having a grammage of between 20 and 100 g/m², may be mentioned as an example, which is in no way limiting, of a material laminate according to the invention. At least the first material layer 2, and preferably both the layers 2, 3, include thermoplastic material. Suitable thermoplastic materials are polyolefins such as polyethylene and polypropene, and polyamides, polyesters and the like. Different types of so-called bicomponent fibres can also be used.

The two material layers 2, 3 are connected to each other by a large number of bonding sites 4. In this connection, the bonding sites 4 are virtually punctate and have been formed by simultaneously compressing the material laminate 1 and supplying energy to it. This has caused the thermoplastic material to soften or melt at the bonding sites 4 and thereby bond together the two layers 2, 3 which are included in the material laminate 1. The bonding together of the first and second material layers 2, 3 is expediently performed by means of heat bonding or by means of ultrasound bonding. The bonding sites 4 are arranged in groups 5 with four bonding sites 4 in each group 5. In this case, the four bonds are located so that they form the corners of a square. The relative distance between the bonding sites 4 in each group is less than the relative distance between the groups 5. In this context, the distances within the groups 5 are determined as being the shortest distance between the bonding sites 4 which are lying adjacent to each other. In a corresponding manner, the distance between the groups 5 is determined as being the shortest distance between groups 5 which are lying adjacent to each other. In both cases, the distances are measured from the edges of the bonding sites 4. The shortest distance between adjacent groups, as measured between the bonding sites 4, in each respective group 5, which are located closest to each other, is preferably 2-6 mm, and the greatest distance between the bonding sites 4 which are located adjacent to each other within the groups is preferably 0.5-1 mm. The former distance is then at least approx. twice as great as the latter distance.

When the melted or softened thermoplastic material in the laminate 1 cools, it solidifies and serves as a bonding agent for the material laminate. In addition to the bonding together of the two material layers 2, 3, a permanent compaction or condensation of the porous structure in the material layers 2, 3 is obtained in this manner. That which is most apparent is the compaction at the actual bonding sites 4. In addition, the particular location of the bonding sites 4 results in the bonded material laminate 1 exhibiting square areas 6 which are enclosed by the bonding site 4 in the groups 5 and which exhibit a higher degree of compaction than do the areas 7 between the groups 5.

The material laminate 1 shown in Figures 1 and 2 is bonded together in such a manner that through-holes 8 have been formed in the first material layer 2 at the bonding sites 4. In addition, the material within and immediately around the bonding sites 4 is strongly compacted, with finer capillaries than the surrounding material. This results in the bonding sites constituting areas which have an increased ability to allow liquid from the first material layer 2 to pass through into the second material 3.

Even if the material laminate 1 is shown with through-holes 8 in the first material layer 2, such a design is not necessary for the invention. Thus, material laminates in which the bonding sites 4 exhibit a surface of a more or less liquid-impermeable nature, or material laminates having both through-holes and liquid-impermeable bonds, are also encompassed. Bonding sites exhibiting low or no liquid permeability are obtained, for example, if the material laminate contains a high proportion of thermoplastic material which has been melted and then allowed to solidify to form a film-like surface. Even if the actual bonding sites 4 are themselves almost completely liquid-impermeable, the compacted fibre structure which has arisen around the bonding sites 4 due to the compression which takes place in connection with the bonding results in the area immediately around each bonding site 4 nevertheless exhibiting a very high ability to transfer liquid.

Furthermore, the compacted areas 6 inside the bonding sites 4 in each group 5 of bonding sites constitute zones possessing an increased ability to transfer liquid. Due to the fact that the distance between the bonding sites 4 within each group 5 is relatively small and preferably from 0.5 mm to 1 mm, the compression in the bonding sites 4 results in the area 6 inside the bonding sites 4 also being affected such that a denser structure is obtained. Thus, the capillary size in the compacted areas 6 which are delimited by the bonding sites 4 is on average less than in areas of the material laminate 1 which are situated between the groups 5 of bonding sites 4. This means that the material laminate 1 exhibits an ability to transfer liquid which is very high in relation to the combined surface of the bonding sites 4. The combined bonded surface preferably constitutes 3-11% of the total surface. The surprisingly good ability to transport and transfer liquid is due to the fact that it is not only the bonding sites 4 themselves and the areas immediately adjacent to the bonding sites which exhibit an increased ability to transfer liquid; the areas which are located between the bonding sites 4 in a group 5 also contribute to the improved liquid transfer.

It is thus possible, by means of the invention, to create areas of greater density and, as a result, increased ability to transport liquid but nevertheless retain high bulk, pliancy and flexibility in the material laminate 1.

Figure 3 shows a bonding pattern for a material laminate 1 according to the invention. The bonding pattern consists of rhombic bonding sites 4 arranged in groups 5' of four bonding sites 4 in each group 5'. In addition, the bonding pattern in Figure 3 exhibits superordinate group formations 5" of four groups 5' having in each case four bonding sites 4. Three different types of areas 6, 7, 9, with different relative material densities, can thus be identified in the bonding pattern in Figure 3. In this case, the densest material structure, with the smallest pore size, is to be found within the groups 5' consisting of four bonding sites 4. The areas 7 of somewhat lower density, and as a result somewhat greater pore size, are to be found in the superordinate group formations 5" of groups 5' having in each case four bonding sites 4. Finally, the least dense areas 9 are to be found between the superordinate group formations 5" and between the superordinate group formations 5" and individual groups 5 of bonding sites 4 which are arranged between the superordinate group formations 5".

Figure 4 shows bonding sites 4 in the form of short (1-1.5 mm) dash-shaped bonds which are arranged in what are in the main parallel bands 5 having a relative distance between the bands which exceeds the distance between the bonding sites 4 which are included in the bands. Within the bands, compacted areas 6 are present between the bonding sites 4, which compacted areas exhibit a smaller pore size than areas 7, which are located between the bands 5.

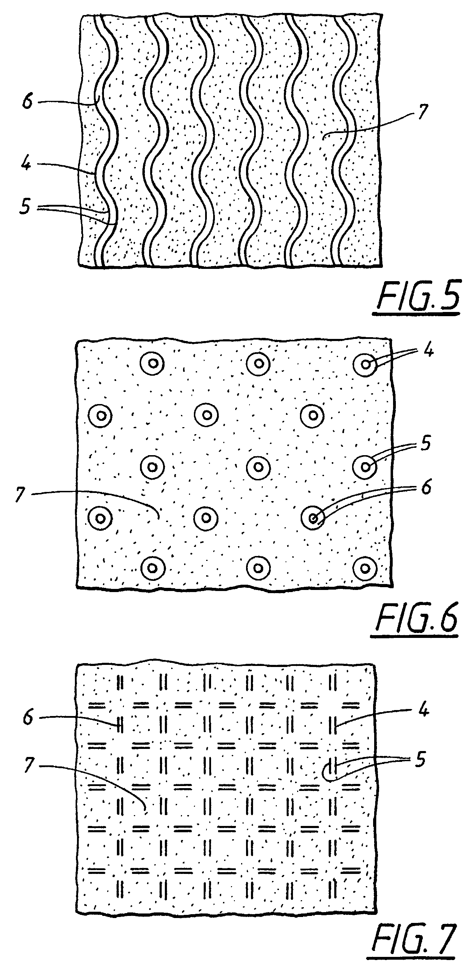

Further utilisable bonding patterns are shown in Figures 5-7, with Figure 5 showing undulating bonding lines 4 which are in the main parallel and which are arranged in pairs with a relative distance between the bonding lines 4 in each pair 5 which exceeds the distance between the pairs 5 of bonding lines 4. The bonding pattern shown in Figure 5 thus results in a material laminate having compacted liquid-transferring areas between the bonding lines 4 in each pair and bulky, distance-creating, soft and airy areas 7 between the bonding pairs 5.

An advantage of arranging the bonding sites 4 in the form of bands or lines is that a surface material having such a bonding pattern in the main conducts liquid in along the bands or lines and counteracts the spread of liquid perpendicularly to the bands or lines. This circumstance can advantageously be exploited in order to decrease the risk of an absorbent product leaking from its edges.

Figure 6 shows a pattern with groups 5 which each consist of two bonding sites 4 in the form of concentric rings which delimit compacted areas 6, while areas 7 of lower density are to be found outside the outer of the annular bonding sites 4.

Figure 7 shows a pattern of short parallel bonding lines 4 which are arranged in pairs at a relative distance such that compacted areas 6 are formed between the bonding lines 4 in each pair 5 and less dense areas are formed between the pairs of bonding lines 4.

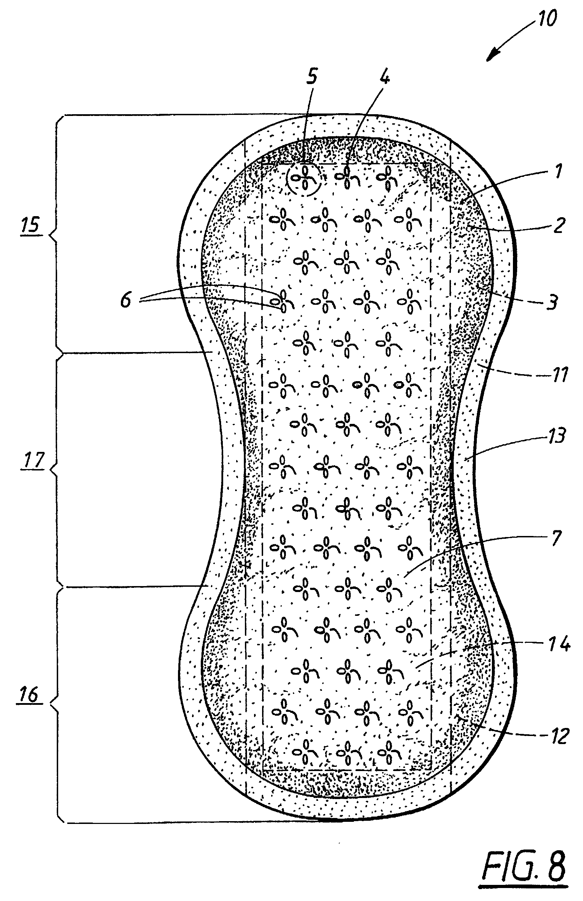

The incontinence shield 10 shown in Figure 8 includes a material laminate 1 according to the invention, which laminate includes a liquid-permeable outer layer 2 and a liquid-permeable liquid-transferring layer 3. Together with a liquid-impermeable outer layer 11, the liquid-permeable outer layer 2 encloses an absorptive body 12. The two outer layers 2, 11 have somewhat larger dimensions in the plane than does the absorptive body 12 and extend some distance beyond the edges of the absorptive body. The outer layers 2, 11 are mutually connected within the projecting parts 13, for example by gluing or welding with heat or ultrasound.

The absorptive body 12 can be of any conventional type whatever. Examples of commonly occurring absorptive materials are cellulose fluff pulp, tissue layers, highly absorbent polymers (so-called superabsorbents), absorbent foam materials, absorbent nonwoven materials and the like. It is normal to combine cellulose fluff pulp and superabsorbents in an absorptive body. It is also normal to use absorptive bodies which are constructed of layers of different materials having different properties as regards the ability to receive, spread and store liquid. This is well known to the skilled person in the field and does not therefore need to be described in detail. The thin absorptive bodies which are nowadays common in, for example, babies' nappies and incontinence shields often consist of a compressed, mixed or layered structure composed of cellulose fluff pulp and superabsorbent.

An attachment member 14, in the form of a longitudinal area of self-adhesive glue, is arranged on the outside of the liquid-impermeable outer layer 11. Before use, the glue area 14 is expediently covered with a detachable protective layer, which is not shown on the drawing, of release agent-treated paper or plastic film. While the attachment member 14 on the depicted incontinence shield consists of a longitudinal glue area, it is naturally possible to conceive of a number of other glue patterns as well as other types of attachment members such as hook-and-loop members, press studs, girdles, special underpants, or the like.

An incontinence shield 10 of the type shown in Figure 8 is first of all intended to be used by individuals who are suffering from relatively mild incontinence problems and is readily accommodated inside a normal pair of underpants. In this connection, the attachment element 14 serves to hold the incontinence shield in place in the underpants during use.

The incontinence shield 10 is hourglass-shaped with wider end parts 15, 16 and a narrower crotch part 17 which is located between the end parts 15, 16. The crotch part 17 is that part of the incontinence shield which is intended, during use, to be to the crotch of the user and to serve as the surface for receiving the excreted body fluid.

As has been previously mentioned, a porous and resilient liquid-transferring layer 3, for example a fibre wad, a porous foam layer, or another of the materials which have been specified as being suitable for the second material layer in the material laminate shown in Figures 1 and 2, is arranged between the liquid-permeable outer layer 2 and the absorptive body 11. The liquid-transferring layer 3 receives the liquid which passes through the outer layer 2. Urination often involves relatively large quantities of liquid which are emitted over a short period. It is therefore essential that the contact between the liquid-permeable outer layer and the liquid-transferring layer 3 which lies inside it is such that the liquid penetrates rapidly into the liquid-transferring layer 3. Due to the fact that the liquid-transferring layer is a layer having a high bulk and a thickness which is preferably from 0.5 mm to 4 mm, the layer 3 can function as a temporary reservoir for the liquid before it is gradually absorbed into the absorptive body 11.

While the liquid-transferring layer 3 is somewhat narrower than the absorptive body 11 in the example shown, it extends over the whole length of the incontinence shield. Such a design is advantageous since it allows some saving of material. It is naturally possible to save further material by not allowing the liquid-transferring layer 3 to extend over the whole of the length of the incontinence shield. For example it is conceivable only to arrange the liquid-transferring layer 3 at the crotch part 17 of the incontinence shield since the majority of the body fluid which is to be absorbed by the incontinence shield can be expected to strike the shield within this part 17.

Commonly employed liquid-transferring layers are often very porous and thereby exhibit a relatively large effective median pore size which is often larger than the effective median pore size of conventional liquid-permeable surface layer materials. The effective median pore size of a fibre material can be measured using a measuring method which is described in EP-A-0,470,392. Since, as a result of the capillary effect, liquid endeavours to pass from wider to finer capillaries and not the other way round, liquid tends to remain in the fibre network of the surface material instead of being drained by the more porous liquid-transferring layer. This means that there is a risk of liquid running on the surface of the outer layer and giving rise to leakage. In addition, liquid remains in the fibre structure of the outer layer as a result of which the surface of the outer layer is felt by the user to be wet and uncomfortable.

Connecting the liquid-permeable outer layer 2 with the liquid-transferring layer 3 as described in connection with the material laminate 1 shown in Figures 1 and 2 results in the liquid-transferring layer 3 being compressed at the bonding site 4. In this way, the liquid-transferring layer 3 exhibits a density gradient, with the density increasing in the direction towards each respective bonding site 4. As a result, the liquid-transferring layer 3 comes to possess a pore size gradient around the bonding sites 4 and an area in which the effective median pore size is less than the median pore size of the liquid-permeable outer layer 2. By grouping the bonding sites 4 in accordance with the invention, it is possible to increase the proportion of the surface of the outer layer laminate 1 in which the median pore size of the liquid-transferring layer 3 is less than the median pore size of the liquid-permeable outer layer 2.

Because of this, the liquid-transferring layer 3 can efficiently drain the outer layer 2 of liquid. As a result of the outer layer 2 being drained of liquid in the area around each respective bonding site 4 and in the intermediate, denser areas 6 between the bonding sites 4 in each group 5 of bonding sites, a deficit of liquid arises in these areas, whereupon an equalisation of liquid will take place with surrounding areas. As a result, the outer layer 2 will come to contain less liquid overall and thereby be felt to be drier against the skin.

By arranging the bonding sites 4 in groups 5 with bond-free, condensed areas 6 between the bonding sites 4, it is thus possible, with a relatively small number of bonds, to obtain very good liquid transport from the liquid-permeable outer layer 2 to the liquid-transferring layer 3. In addition, bond-free areas 7 are left between the groups 5, thereby imparting an undulating structure to the surface of the incontinence shield 10 which is facing the user. In addition, the bond-free areas 7 between the bonding groups 5 are bulky and soft and result in the material laminate 1 being airy and comfortable while at the same time providing a good distancing effect, as a result of which the skin of the user can be kept dry even after wetting.

In order to obtain good liquid transfer between the liquid-transferring layer 3 and the absorptive body 11, the absorptive body should have a greater liquid affinity than the liquid-transferring layer 3. This can be achieved, for example, by the liquid-transferring layer 3 being less hydrophilic than the absorptive body 11 and/or by the absorptive body 11 having more of a fine-capillary structure than the liquid-transferring layer 3.

The invention is not to be regarded as being limited to the embodiment examples which are described in this present document; on the contrary, it is possible to conceive of a number of further variants and modifications within the scope of the subsequent patent claims.

## Claims

1. A material laminate (1) with a planar dimension and a thickness direction perpendicular to the planar dimension, including a first liquid-permeable fibrous material layer (2) and a second liquid-permeable, porous and resilient material layer (3), with at least one of the material layers (2, 3) including thermoplastic material and the two material layers (2, 3) being mutually connected by the material laminate (1) exhibiting bonding sites (4) within which the thermoplastic material has been caused to at least partially soften or melt and thereby bond together the two material layers (2, 3), wherein the bonding areas extend in the thickness direction of the material laminate (1) through the first material layer (2) and at least through a part of the second material layer (3), **characterized in that** said bonding areas are arranged in two or more groups (5) with at least two bonding sites (4) in each group (5), with the greatest relative distance between two bonding sites (4), which are situated adjacent to each other, in a particular group (5) being less than the shortest distance between the group (5) and its closest adjacent group (5), as a result of which the material laminate (1) exhibits bond-free areas (6) between the bonding sites (4) within each bonding group (5) which have a higher density than bond-free areas (7, 9) of the material laminate which are situated between the bonding groups (5), and wherein the shortest relative distance x between two groups (5) of bonding sites (4), which two groups are situated adjacent to each other, is at least twice as great as the greatest relative distance y between two bonding sites (4) which are arranged adjacent to each other within the groups (5).

2. A material laminate according to Claim 1, **characterized in that** the bonding sites (4) comprise point bonds.

3. A material laminate according to Claim 1 or 2, **characterized in that** the bonding sites (4) comprise bonding lines.

4. A material laminate according to Claim 1, 2 or 3, **characterized in that** the bonding sites (4) comprise rectangular bonds.

5. A material laminate according to any one of Claims 1-4, **characterized in that** the bonding sites comprise circular bonds.

6. A material laminate according to any one of the preceding claims, **characterized in that** the first material layer (2) exhibits through-holes at the bonding sites (4).

7. A material laminate according to any one of the preceding claims, **characterized in that** the first material layer (2) consists of a nonwoven material.

8. A material laminate according to Claim 7, **characterized in that** the nonwoven material is a carded, thermally bonded material.

9. A material laminate according to any one of the preceding claims, **characterized in that** the second material layer (3) is a fibre wad layer having a thickness of 0.5-4 mm.

10. A material laminate according to any one of the preceding claims, **characterized in that** the ratio x/y between the distances x and y is from 2/1 to 12/1.

11. A material laminate according to any one of the preceding claims, **characterized in that** x is 2-6 mm and y is 0.5-1 mm.

12. An absorbent product including a liquid-permeable outer layer (2), a liquid-impermeable outer layer (11) and an absorptive body (12) enclosed between the two outer layers (2, 11), and also a liquid-permeable liquid-transferring layer (3) arranged between the liquid-permeable outer layer (2) and the absorptive body (12), **characterized in that** the liquid-permeable outer layer (2) and the liquid-permeable liquid-transferring layer (3) are present in the form of a material laminate in accordance with any one of the preceding patent claims.

## Patentansprüche

1. Materiallaminat (1) mit einer ebenen Abmessung und einer Dickenrichtung senkrecht zu der ebenen Abmessung, mit einer ersten flüssigkeitsdurchlässigen faserigen Materialschicht (2) und einer zweiten flüssigkeitsdurchlässigen, porösen und nachgiebigen Materialschicht (3), wobei wenigstens eine der Materialschichten (2, 3) ein thermoplastisches Material aufweist, und die beiden Materialschichten (2, 3) dadurch miteinander verbunden sind, dass das Materiallaminat (1) Verbindungsstellen (4) aufweist, innerhalb welcher das thermoplastische Material veranlasst wurde, sich wenigstens teilweise aufzuweichen oder zu schmelzen und hierdurch die beiden Materialschichten (2, 3) miteinander zu verbinden, wobei die Verbindungsbereiche sich in der Dickenrichtung des Materiallaminats (1) durch die erste Materialschicht (2) und wenigstens durch einen Teil der zweiten Materialschicht (3) erstrecken, **dadurch gekennzeichnet, dass** die Verbindungsbereiche in zwei oder mehr Gruppen (5) angeordnet sind, mit wenigstens zwei Verbindungsstellen (4) in jeder Gruppe (5), wobei der größte relative Abstand zwischen zwei Verbindungsstellen (4), die benachbart zueinander angeordnet sind, in einer bestimmten Gruppe (5) geringer ist als der kürzeste Abstand zwischen der Gruppe (5) und ihrer nächsten benachbarten Gruppe (5), so dass als Ergebnis das Materiallaminat (1) verbindungsfreie Bereiche (6) zwischen den Verbindungsstellen (4) innerhalb jeder Verbindungsgruppe (5) aufweist, die eine höhere Dichte aufweisen als verbindungsfreie Bereiche (7, 9) des Materiallaminats, die zwischen den Verbindungsgruppen (5) angeordnet sind, und wobei der kürzeste relative Abstand x zwischen zwei Gruppen (5) von Verbindungsstellen (4), welche zwei Gruppen benachbart zueinander angeordnet sind, wenigstens zweimal so groß wie der größte relative Abstand y zwischen zwei Verbindungsstellen (4) ist, die benachbart zueinander innerhalb der Gruppen (5) angeordnet sind.

2. Materiallaminat nach Anspruch 1, **dadurch gekennzeichnet, dass** die Verbindungsstellen (4) Punktverbindungen aufweisen.

3. Materiallaminat nach Anspruch 1 oder 2, **dadurch gekennzeichnet, dass** die Verbindungsstellen (4) Verbindungslinien aufweisen.

4. Materiallaminat nach einem der Ansprüche 1, 2 oder 3, **dadurch gekennzeichnet, dass** die Verbindungsstellen (4) rechtwinklige Verbindungen aufweisen.

5. Materiallaminat nach einem der Ansprüche 1 bis 4, **dadurch gekennzeichnet, dass** die Verbindungsstellen kreisförmige Verbindungen aufweisen.

6. Materiallaminat nach einem der vorangehenden Ansprüche, **dadurch gekennzeichnet, dass** die erste Materialschicht (2) an den Verbindungsstellen (4) durchgehende Öffnungen aufweist.

7. Materiallaminat nach einem der vorangehenden Ansprüche, **dadurch gekennzeichnet, dass** die erste Materialschicht (2) aus einem Vliesmaterial besteht.

8. Materillaminat nach Anspruch 7, **dadurch gekennzeichnet, dass** das Vliesmaterial ein kardiertes, thermisch verbundenes Material ist.

9. Materiallaminat nach einem der vorangehenden Ansprüche, **dadurch gekennzeichnet, dass** die zweite Materialschicht (3) eine Faserwatteschicht mit einer Dicke von 0,5-4 mm ist.

10. Materiallaminat nach einem der vorangehenden Ansprüche, **dadurch gekennzeichnet, dass** das Verhältnis x/y zwischen den Abständen x und y von 2/1 bis 12/1 beträgt.

11. Materiallaminat nach einem der vorangehenden Ansprüche, **dadurch gekennzeichnet, dass** x 2-6 mm und y 0,5-1 mm ist.

12. Absorptionsprodukt mit einer flüssigkeitsdurchlässigen Außenschicht (2) und einer flüssigkeitsundurchlässigen Außensschicht (11) und einem Absorptionskörper (12), der zwischen den beiden Außenschichten (2, 11) eingeschlossen ist, und ferner mit einer flüssigkeitsdurchlässigen Flüssigkeitsübertragungsschicht (3), die zwischen der flüssigkeitsdurchlässigen Außenschicht (2) und dem Absorptionskörper (12) angeordnet ist, **dadurch gekennzeichnet, dass** die flüssigkeitsdurchlässige Außenschicht (2) und die flüssigkeitsdurchlässige Flüssigkeitsübertragungsschicht (3) in der Form eines Materiallaminats gemäß einem der vorangehenden Patentansprüche vorliegen.

## Revendications

1. Stratifié (1) de matériaux, comportant une dimension plane et un sens de l'épaisseur, perpendiculaire à la dimension plane, comprenant une première couche (2) de matériau fibreux perméable aux liquides et une deuxième couche (3) de matériau perméable aux liquides, poreux et élastique, au moins une des couches (2, 3) de matériau comprenant un matériau thermoplastique et les deux couches (2, 3) de matériaux étant mutuellement connectées par le stratifié (1) de matériaux présentant des emplacements de liaison (4) dans lesquels on a fait au moins partiellement ramollir ou fondre le matériau thermoplastique et, de ce fait, on l'a lié aux deux couches (2, 3) de matériau, les zones de liaison s'étendant dans le sens de l'épaisseur du stratifié (1) de matériaux en traversant la première couche (2) de matériau et en traversant au moins partiellement une partie de la deuxième couche (3) de matériau, **caractérisé en ce que** lesdites zones de liaison sont disposées en deux ou plusieurs groupes (5), avec au moins deux emplacements de liaison (4) dans chaque groupe (5), la plus grande distance relative entre deux emplacements de liaison (4), situés de façon adjacente l'un à l'autre, dans un groupe (5) particulier, étant inférieure à la plus courte distance entre un groupe (5) et le groupe (5) qui lui est le plus proche, en résultat de quoi le stratifié (1) de matériaux présente entre les emplacements de liaison (4) dans chaque groupe de liaison (5) des zones (6) exemptes de liaisons qui ont une masse volumique supérieure à celle des zones (7, 9) exemptes de liaisons du stratifié de matériaux qui sont situées entre les groupes de liaison (5), et la distance relative la plus faible x entre deux groupes (5) d'emplacements de liaison (4), lesquels deux groupes sont situés de façon adjacente l'un à l'autre, étant au moins deux fois plus grande que la distance relative la plus grande y entre deux emplacements de liaison (4) qui sont disposés de façon adjacente l'un à l'autre dans les groupes (5).

2. Stratifié de matériaux selon la revendication 1, **caractérisé en ce que** les emplacements de liaison (4) comprennent des liaisons ponctuelles.

3. Stratifié de matériaux selon la revendication 1 ou 2, **caractérisé en ce que** les emplacements de liaison (4) sont constitués par des lignes de liaison.

4. Stratifié de matériaux selon la revendication 1, 2 ou 3, **caractérisé en ce que** les emplacements de liaison (4) sont constitués par des liaisons de forme rectangulaire.

5. Stratifié de matériaux selon l'une quelconque des revendications 1 à 4, **caractérisé en ce que** les emplacements de liaison sont constitués par des liaisons de forme circulaire.

6. Stratifié de matériaux selon l'une quelconque des revendications qui précèdent, **caractérisé en ce que** la première couche (2) de matériau présente des trous traversants à l'emplacement des emplacements de liaison (4).

7. Stratifié de matériaux selon l'une quelconque des revendications qui précèdent, **caractérisé en ce que** la première couche (2) de matériau consiste en un matériau non tissé.

8. Stratifié de matériaux selon la revendication 7, **caractérisé en ce que** le matériau non tissé est un matériau cardé, lié thermiquement.

9. Stratifié de matériaux selon la revendication 7, **caractérisé en ce que** la deuxième couche (3) de matériau est une couche de ouate de fibres ayant une épaisseur comprise entre 0,5 et 4 mm.

10. Stratifié de matériaux selon l'une quelconque des revendications qui précèdent, **caractérisé en ce que** le rapport x/y entre les distances x et y est compris entre 2/1 et 12/1.

11. Stratifié de matériaux selon l'une quelconque des revendications qui précèdent, **caractérisé en ce que** x est compris entre 2 et 6 mm et y est compris entre 0,5 et 1 mm.

12. Produit absorbant comprenant une couche extérieure (2) perméable aux liquides, une couche extérieure (11) imperméable aux liquides et un corps absorbant (12) enfermé entre les deux couches extérieures (2, 11), ainsi qu'une couche (3) de transfert de liquides perméable aux liquides, disposée entre la couche extérieure (2) perméable aux liquides et le corps absorbant (12), **caractérisé en ce que** la couche extérieure (2) perméable aux liquides et la couche (3) de transfert de liquides perméable aux liquides se présente sous la forme d'un stratifié de matériaux selon l'une quelconque des revendications qui précèdent.
